# EUROPÄISCHE PATENTSCHRIFT

(11) **EP 2 621 424 B1**
(45) Veröffentlichungstag und Bekanntmachung des Hinweises auf die Patenterteilung: **07.11.2018**
(21) Anmeldenummer: 11805751.2
(22) Anmeldetag: 24.09.2011
(51) Int. Cl.: A61F 9/007

(54) **STEUERUNGSVORRICHTUNG FÜR EIN OPHTHALMOCHIRURGISCHES SYSTEM**
CONTROL DEVICE FOR AN OPHTHALMIC SURGICAL SYSTEM
DISPOSITIF DE COMMANDE D'UN SYSTÈME DE CHIRURGIE OPHTALMOLOGIQUE

(30) Priorität: 30.09.2010 DE 102010047011
(43) Veröffentlichungstag der Anmeldung: 07.08.2013
(73) Patentinhaber: Carl Zeiss Meditec AG, 07745 Jena (DE)
(72) Erfinder: KRAUS, Martin, 73460 Hüttlingen (DE); HAUGER, Christoph, 73431 Aalen (DE)
(74) Vertreter: Carl Zeiss AG - Patentabteilung
(86) Internationale Anmeldenummer: PCT/DE2011/001776
(87) Internationale Veröffentlichungsnummer: WO 2012/041289

(56) Entgegenhaltungen:
- WO-A1-2007/121144
- US-A1- 2008 319 451
- US-A1- 2009 306 581

## Beschreibung

Die Erfindung betrifft eine Steuerungsvorrichtung für ein ophthalmochirurgisches System zur Phakoemulsifikation einer Augenlinse und ein ophthalmochirurgisches System mit einer solchen Steuerungsvorrichtung.

Zur Behandlung einer Augenlinsentrübung, welche in der Medizin als Grauer Star bezeichnet wird, gibt es mehrere chirurgische Techniken. Die am weitesten verbreitete Technik ist die Phakoemulsifikation, bei der eine dünne Nadel in die erkrankte Linse eingeführt und mit Ultraschall zu Schwingungen angeregt wird. Die vibrierende Nadel emulsifiziert in ihrer nächsten Umgebung die Linse derart, dass die entstehenden Linsenpartikel durch eine Leitung mittels einer Pumpe abgesaugt werden können. Dabei wird ein Spülfluid (Irrigationsfluid) zugeführt, wobei das Absaugen der Partikel und des Fluides durch eine Aspirationsleitung erfolgt, welche üblicherweise innerhalb der Nadel angeordnet ist. Ist die Linse vollständig emulsifiziert und entfernt worden, kann in den leeren Kapselsack eine neue künstliche Linse eingesetzt werden, so dass ein derart behandelter Patient wieder ein gutes Sehvermögen erreichen kann.

Eine Operation des Grauen Stars bzw. eine Kataraktoperation ist ein Eingriff mit einer relativ geringen Komplikationsrate und großer Patientenzahl. In Deutschland werden etwa 600.000 Operationen pro Jahr durchgeführt, wobei die getrübte Linse durch ein künstliches Linsenimplantat ersetzt wird. Die relativ geringe Komplikationsrate lässt sich jedoch nur erreichen, wenn ein Operateur mit viel Erfahrung die Operation durchführt. Bei der Zerkleinerung der Augenlinse durch eine mit Ultraschall schwingende Nadelspitze ist es unvermeidbar, dass sich während der Operation ein relativ großes Partikel so vor eine Nadelspitze platziert, dass die Nadelspitze bzw. ihre Absaugöffnung verstopft wird. Dieser Zustand wird als Okklusion bezeichnet. In einem solchen Fall steigt der Ansaugdruck innerhalb der Aspirationsleitung deutlich an, wobei eine Emulsifikation in diesem Moment unterbrochen ist. Erst wenn zum Beispiel durch einen sehr starken Energieeintrag, einen deutlichen Anstieg des Ansaugdruckes, oder durch eine Umkehrung der Aspirationspumpenlaufrichtung das Partikel von der Nadelspitze wieder entfernt ist, kann ein übliches Absaugen des Fluides und der kleinen Partikel erfolgen. In einem solchen Moment wird eine Verstopfung somit aufgebrochen, wobei der zuvor anliegende hohe Unterdruck schlagartig abnimmt. Der dadurch entstehende Sog kann dazu führen, dass nicht nur kleine Partikel und Fluid zur Aspirationsnadel gezogen werden, sondern auch ein Teil des Kapselsackes mit der Nadel in Kontakt kommt. Wenn der Kapselsack durchstochen wird, führt dies zu erheblichen Komplikationen für den Patienten, welche unbedingt vermieden werden müssen. Ein erfahrener Operateur hat im Laufe der Zeit ein Gespür dafür entwickelt, wenn eine Okklusion kurz bevorsteht, aufzubrechen. Trotzdem bleibt stets ein Risiko, dass eine Verletzung des Patientenauges auftreten könnte.

US 2009/0306581 A1 zeigt ein ophthalmochirurgisches System, bei dem ein Operationsmikroskop und eine digitale Videokamera vorgesehen sind, um in Echtzeit Multimedia-Daten der Operation bereitzustellen.

US 2008/0319451 A1 zeigt ein ophthalmochirurgisches System, bei dem am distalen Ende der Aspirationsleitung ein Drucksensor vorgesehen ist, um eine Okklusion zu detektieren.

Es ist eine Aufgabe der Erfindung, eine Steuerungsvorrichtung für ein ophthalmochirurgisches System zur Phakoemulsifikation einer Augenlinse zu schaffen, bei dem das Auftreten einer Okklusion einer Phakoemulsifikationsnadel und das Aufbrechen einer derartigen Okklusion an einer solchen Nadel schnell und zuverlässig erkannt werden kann, so dass ein Risiko für eine Verletzung eines Patientenauges gering ist. Ferner ist es eine Aufgabe, ein ophthalmochirurgisches System mit einer derartigen Steuerungsvorrichtung zu schaffen.

Die Aufgabe wird für die Steuerungsvorrichtung durch den Gegenstand des unabhängigen Patentanspruchs 1 gelöst. Vorteilhafte Weiterbildungen der Erfindung sind Gegenstand der Unteransprüche. Die Aufgabe für das ophthalmochirurgische System wird durch den Gegenstand des unabhängigen Patentanspruchs 11 gelöst.

Die Steuerungsvorrichtung für ein ophthalmochirurgisches System zur Phakoemulsifikation einer Augenlinse weist auf:
- ein optisches System, mit dem von einem Objektbereich ein Bild erzeugbar ist, wobei sich in dem Objektbereich mindestens ein Teil der zu emulsifizierenden Augenlinse und ein Teil einer Nadel eines Phakohandstückes mit einer Aspirationsleitung anordnen lässt,
- eine Bildauswertereinheit, welche geeignet ist, das erzeugte Bild so auszuwerten, dass mindestens eine von einer Okklusion der Aspirationsleitung abhängige Auswertegröße ermittelt wird, welche eine kinematische Messgröße und/oder eine geometrische Messgröße und/oder eine optische Messgröße eines durch Emulsifikation erzeugten Partikels (21, 31) der zu behandelnden Augenlinse (1) ist, und
- eine Steuereinheit, mit der in Abhängigkeit von der mindestens einer zugeführten Auswertegröße eine Steuergröße ermittelbar ist, wobei sich mittels der Steuergröße ein Betrag eines Parameters des ophthalmochirurgischen Systems steuern lässt.

Ein Parameter des ophthalmochirurgischen Systems wird somit durch eine Steuergröße gesteuert, welche in Abhängigkeit von einer Auswertegröße steht, die wiederum von einer Okklusion in der Aspirationsleitung abhängig ist. Diese Auswertegröße wird mittels eines Bildes ermittelt, welches von einem optischen System erzeugt wird. Durch den Einsatz eines optischen Systems ist eine sehr schnelle Bildauswertung möglich.

Gemäß dem Stand der Technik wird eine Okklusion erkannt, indem ein Druck in der Aspirationsleitung aufgenommen wird. Bis eine Druckänderung an einer Nadel zu einem hinter der Nadel oder gar dem Phakohandstück angeordneten Drucksensor in einer Konsole eines ophthalmochirurgischen Systems gelangt, kann relativ viel Zeit vergehen. Eine dann initiierte Reaktion des ophthalmochirurgischen Systems auf die detektierte Druckänderung kann oftmals zu spät eintreten, so dass eine Verletzung des Patientenauges nicht vermieden werden kann. Eine Bildauswertung mittels eines optischen Systems erfordert kein träges Aufbauen eines Druckunterschiedes. Mit der erfindungsgemäßen Steuerungsvorrichtung ist es nun möglich, schneller als bisher das Eintreten einer Okklusion und das Aufbrechen einer Okklusion zu erfassen. Bei einem Okklusionsdurchbruch ist es somit möglich, einen Parameter des ophthalmochirurgischen Systems schneller anzusteuern und das Risiko für eine Verletzung des Patientenauges zu reduzieren.

Gemäß der Erfindung ist die mindestens eine Auswertegröße der Bildauswerteeinheit eine kinematische Messgröße und/oder eine geometrische Messgröße und/oder eine optische Messgröße eines durch Emulsifikation erzeugten Partikels der zu behandelnden Augenlinse. Bei der kinematischen Messgröße kann es sich dabei um eine Geschwindigkeit oder Beschleunigung des Partikels während einer vorbestimmten Zeitdauer handeln. Bei dem von dem optischen System aufgenommenen Bild wird mittels der Bildauswerteeinheit somit ein Partikel erfasst, wobei die Geschwindigkeit oder die Beschleunigung dieses Partikels eine Aussage darüber erlaubt, ob eine Okklusion vorliegt oder nicht. Bei einer sehr geringen Geschwindigkeit oder Beschleunigung ist das Partikel nahezu oder vollständig in Ruhe und liegt damit an der Nadel an. Damit kann von einer Okklusion ausgegangen werden. Bei einer sehr hohen Geschwindigkeit oder einer hohen Beschleunigung kann davon ausgegangen werden, dass das entsprechende Partikel vor der Nadel zügig in die Ansaugöffnung der Aspirationsleitung gelangt, so dass entweder eine Okklusion aufbricht oder diese noch nicht vorhanden ist, wobei dies von der Vorgeschichte abhängt. Wenn zuvor eine Okklusion ermittelt worden ist, bedeutet eine hohe Geschwindigkeit oder Beschleunigung ein Aufbrechen der Okklusion. Wenn noch keine Okklusion ermittelt worden war, bedeutet eine hohe Geschwindigkeit oder Beschleunigung eines erfassten Partikels vor der Nadel ein zügiges Absaugen, wobei für das Patientenauges noch keine Gefahr besteht.

Alternativ oder zusätzlich kann die mindestens eine Auswertegröße der Bildauswerteeinheit eine geometrische Messgröße eines Partikels der zu emulsifizierenden Augenlinse sein, welches sich in unmittelbarer Nähe der Spitze der Nadel befindet. Eine geometrische Messgröße kann eine Fläche oder einen Umfang oder ein Volumen des zu emulsifizierenden Partikels in dem erzeugten Bild sein. Falls zum Beispiel die Fläche des erfassten Partikels vor einer Spitze der Nadel so klein ist, dass zu erwarten ist, dass auch die engste Stelle innerhalb einer Aspirationsleitung gut passiert werden kann, ohne dass eine Verstopfung auftritt, kann ein Parameter oder können mehrere Parameter des ophthalmochirurgischen Systems so gesteuert sein, dass ein möglichst schnelles Absaugen von Partikeln und Fluid erzielt wird. Falls die Fläche aber größer als zum Beispiel der kleinste Querschnitt einer Aspirationsleitung ist, muss mit einer Verstopfung gerechnet werden, so dass ein Parameter des ophthalmochirurgischen Systems entsprechend angesteuert wird, um zum Beispiel die Okklusion schnell aufbrechen zu können.

Gemäß der Erfindung kann die mindestens eine Auswertegröße eine optische Messgröße eines Partikels der zu emulsifizierenden Augenlinse sein, welches sich in unmittelbarer Nähe der Spitze der Nadel befindet. Eine optische Messgröße kann dabei eine Grauwertintensität oder eine Farbintensität sein. Ein großes Partikel führt in einem auszuwertenden Bild zu einer stärkeren Abschattung als ein kleines Partikel, so dass allein aufgrund einer Grauwertintensität oder Farbintensität in dem aufgenommenen Bild auf die Größe des Partikels und die Wahrscheinlichkeit einer Okklusion geschlossen werden kann.

In Abhängigkeit von der Auswertegröße kann von der Steuerungsvorrichtung gemäß der Erfindung ein Betrag eines Parameters des ophthalmochirurgischen Systems gesteuert werden. Bei einem solchen Parameter des ophthalmochirurgischen Systems kann es sich um eine dem Phakohandstück zugeführte Ultraschallenergie, eine Saugleistung einer Aspirationspumpe oder einen Volumenstrom eines Fluides zur Belüftung einer Aspirationsleitung handeln. Falls erkannt wird, dass eine Okklusion gerade durchbricht, kann eine dem Phakohandstück zugeführte Ultraschallenergie auf einen niedrigen Betrag heruntergesteuert werden. Damit verringert sich der Hub der Nadel, so dass relativ wenig Energie zum Auge zugeführt wird. Der Betrag einer zugeführten Ultraschallenergie kann dann so niedrig gesetzt werden, dass eine weitere Emulsifikation eines Augenlinsenmaterials nicht mehr möglich ist. Alternativ oder zusätzlich kann ein Parameter auch eine Saugleistung einer Aspirationspumpe sein. Sobald ein Durchbruch einer Okklusion erkannt wird, kann durch Verringern der Saugleistung der aufgebaute Unterdruck in der Aspirationsleitung in seiner Höhe vermindert werden. Das schlagartige Absenken des Unterdruckes nach Aufbrechen der Okklusion beginnt dann nicht mehr bei einem so starken Unterdruck. Es ist auch möglich, das in diesem Moment die Förderrichtung der Aspirationspumpe umgekehrt wird, so dass kein Saugen, sondern ein Drücken entsteht. Damit kann die Gefahr deutlich verringert werden, dass aufgrund des starken Unterdruckes zum Beispiel die Linsenkapsel zur Nadel hingezogen und durchstochen wird.

Gemäß einer Weiterbildung der Erfindung kann die mindestens eine Auswertegröße der Bildauswerteeinheit eine geometrische Messgröße eines die Nadel umgebenden Gewebes sein. Die geometrische Messgröße kann zum Beispiel ein Abstand zwischen der Nadel und einem Kapselsack sein. Wenn der Abstand kleiner als ein vorbestimmter Wert ist, kann von der Steuereinheit die Steuergröße so ermittelt werden, dass die dem Handstück zugeführte Ultraschallenergie auf einen minimalen Betrag oder auf den Wert Null gesteuert wird. Zusätzlich kann noch die Aspirationspumpe so gesteuert werden, dass der Unterdruck in der Apsirationsleitung einen niedrigeren Wert erreicht. Damit kann die Gefahr eines Durchstechens eines Kapselsackes aufgrund einer stark schwingenden Nadelspitze oder eines starken Unterdruckes verringert werden.

Das optische System kann ein Lichtmikroskop und/oder ein OCT-System aufweisen. Gemäß einer Weiterbildung der Erfindung ist das optische System mindestens teilweise im Phakohandstück angeordnet. Ist das optische System ein OCT-System, kann dieses OCT-System aufweisen: eine Quelle für zeitlich inkohärentes und räumlich kohärentes Licht, eine Vorrichtung zur Überlagerung von Laserstrahlung, die aufgrund von Streuzentren im Linsengewebe in den Probenstrahlengang zurückgestrahlt wird, mit der Laserstrahlung aus einem Referenzstrahlengang, so dass sich ein Endreferenzsignal und daraus die Position von Streuzentren für die Laserstrahlung ermitteln lässt, und eine Bilderzeugungseinheit, um ein Bild zu erzeugen. Die OCT-Faser kann innerhalb oder an der Nadel angeordnet werden, wobei die Faser rotierend oder scannend ausgelegt sein kann, um einen Volumenbereich unmittelbar vor der Nadel zu erfassen.

Weitere Vorteile und Merkmale der Erfindung werden mit Bezug auf die nachfolgenden Zeichnungen erklärt, in welchen zeigen:
- Figur 1: eine schematische Darstellung eines ophthalmochirurgisches System mit einer Steuerungsvorrichtung gemäß einer ersten Ausführungsform der Erfindung;
- Figur 2: ein Bild einer Bildauswerteeinheit mit einem Teil einer Nadel eines Phakohandstückes und einem relativ kleinen Partikel;
- Figur 3: ein Bild einer Bildauswerteeinheit mit einem Teil einer Nadel eines Phakohandstückes mit einem relativ großen Partikel;
- Figur 4: ein Diagramm mit einem Druckverlauf und ein Diagramm mit einem zugehörigen Geschwindigkeitsverlauf eines Partikels einer Augenlinse vor einer Nadel eines Phakohandstückes in Abhängigkeit von der Zeit;
- Figur 5: ein Flussdiagramm, mit dem der Einfluss einer kinematischen Messgröße als Auswertegröße auf einen zu steuernden Parameter des ophthalmochirurgischen Systems dargestellt ist;
- Figur 6: ein Flussdiagramm, mit dem der Einfluss einer kinematischen Messgröße und einer geometrischen Messgröße eines Partikels auf einen Parameter des ophthalmochirurgischen Systems dargestellt ist; und
- Figur 7: eine schematische Darstellung einer zweiten Ausführungsform eines ophthalmochirurgischen Systems mit einer Steuerungsvorrichtung gemäß der Erfindung.

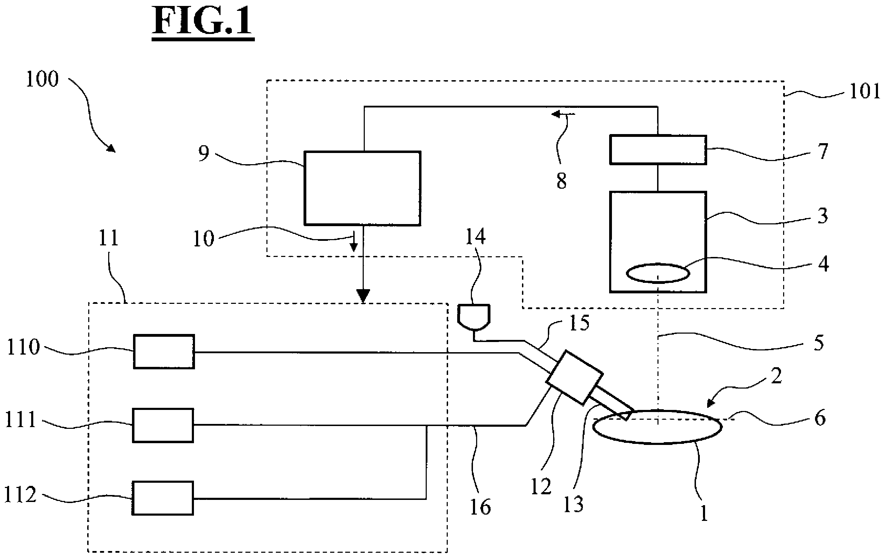

In Figur 1 ist ein ophthalmochirurgisches System 100 mit einer Steuerungsvorrichtung 101 dargestellt. Eine zu emulsifizierende Augenlinse 1 ist in einem Objektbereich 2 eines optischen Systems 3 angeordnet. Das optische System 3 ist bei dieser in Figur 1 dargestellten Ausführungsform ein Lichtmikroskop, wobei ein Hauptobjektiv 4 mit einer optischen Achse 5 und einer Fokusebene 6 vorgesehen ist. Die Fokusebene 6 kann innerhalb eines Objektbereiches 2 eingestellt werden. Das optische System 3 weist eine Bilderzeugungseinheit auf, welche ein Bild erzeugt, das von einer Bildauswerteeinheit 7 ausgewertet werden kann. Von der Bildauswerteeinheit 7 kann eine Auswertegröße 8 ermittelt werden, welche zu einer Steuereinheit 9 zugeführt werden kann. In Abhängigkeit von der Auswertegröße 8 kann die Steuereinheit 9 eine Steuergröße 10 ermitteln, welche auf eine Systemkomponenteneinheit 11 einwirken kann. Die Systemkomponenteneinheit 11 kann eine Energieversorgung 110 zum Bereitstellen einer Ultraschallenergie für die Nadel 13 eines Phakohandstückes 12 aufweisen. Eine Systemkomponenteneinheit 11 kann alternativ oder zusätzlich eine Aspirationspumpe 111 aufweisen, welche Fluid und emulsifizierte Partikel der Augenlinse durch eine Aspirationsleitung 16 ansaugt. Eine Systemkomponenteneinheit 11 kann auch einen Refluxbehälter 112 aufweisen, von dem ein Fluid in die Aspirationsleitung 16 zuführbar ist, um im Falle eines Okklusionsdurchbruches den starken Unterdruck in der Aspirationsleitung 16 zu beeinflussen.

Die Systemkomponenteneinheit 11 ist mit einem Phakohandstück 12 mit einer Nadel 13 gekoppelt, wobei üblicherweise innerhalb der Nadel ein Teil der Aspirationsleitung 16 geführt ist. Ein Irrigationsfluid wird von einem Irrigationsfluidbehälter 14 durch eine Irrigationsfluidleitung 15 zu dem Phakohandstück 12 geleitet, um während der Phakoemulsifikation genügend Irrigationsfluid zur Verfügung zu stellen. Die Systemkomponenteneinheit 11 kann außer der erwähnten Energieversorgung 110, der Aspirationspumpe 111 oder dem Refluxbehälter 112 auch andere Komponenten aufweisen, welche geeignet sind, einen Parameter eines ophthalmochirurgischen Systems zu beeinflussen.

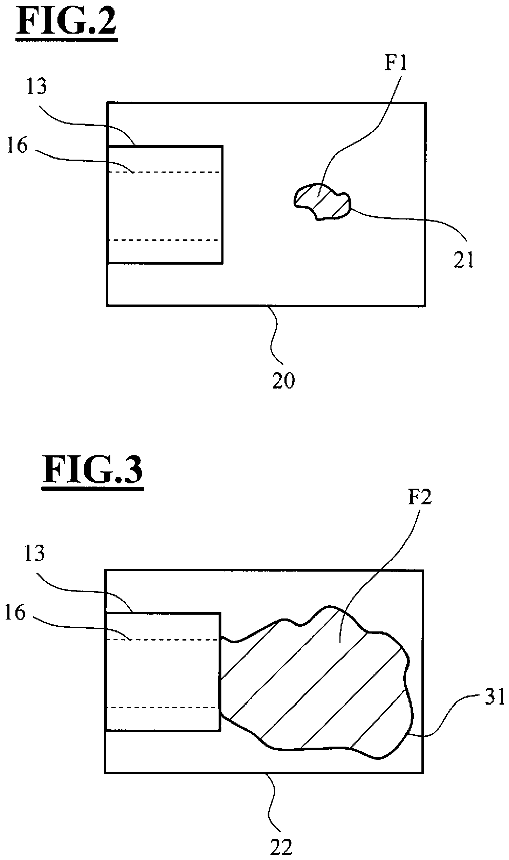

In Figur 2 ist ein Bild 20 eines von dem optischen System 3 erzeugten Bildes dargestellt, wobei dieses Bild 20 einen vorderen Rand einer Nadel 13 des Phakohandstückes 12 zeigt. Innerhalb der Nadel 13 ist eine Aspirationsleitung 16 angeordnet, die an der Spitze der Nadel 13 endet. Zusätzlich ist ein relativ kleines Partikel 21 mit einer Fläche F1 dargestellt. Die Fläche F1 des Partikels 21 ist so klein, dass zu erwarten ist, dass auch der engste Querschnitt der Aspirationsleitung 16 passiert werden kann, ohne dass dieses Partikel in der Aspirationsleitung 16 hängen bleibt. Bei einem sich derart auf eine Nadel 13 zubewegenden Partikel 21 ist nicht zu erwarten, das eine Verstopfung bzw. eine Okklusion der Aspirationsleitung 16 auftritt.

In Figur 3 ist ein Bild 22 einer Bilderzeugungseinheit des optischen Systems 3 dargestellt, wobei das Bild 22 eine Nadel 13 mit einer Aspirationsleitung 16 und ein Partikel 31 zeigt. Das Partikel 31 besitzt eine Fläche F2, welche erwarten lässt, dass es die Aspirationsleitung 16 nicht passieren kann. Somit muss mit einer Verstopfung der Aspirationsleitung 16 gerechnet werden. Wenn von der Bilderzeugungseinheit ein solches Bild 22 erzeugt wird, kann von der Bildauswerteeinheit 7 zum Beispiel durch eine kinematische Messgröße, eine geometrische Messgröße oder eine optische Messgröße erkannt werden, dass ein Betrag eines Parameters des ophthalmochirurgischen Systems anzusteuern ist, so dass die Folgen einer Okklusion oder eines Okklusionsdurchbruches verringert werden.

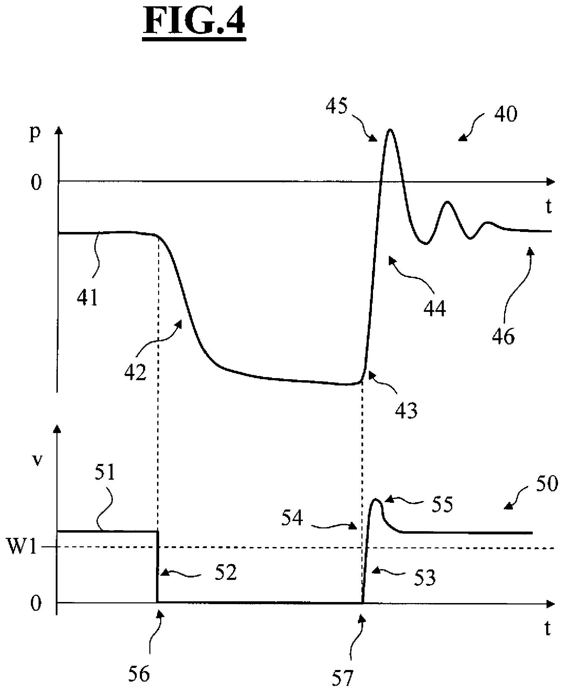

In Figur 4 ist ein Diagramm 40 dargestellt, welches einen Druckverlauf innerhalb einer Aspirationsleitung in Abhängigkeit von der Zeit zeigt. Zu Beginn eines Ansaugvorganges kann ein erster Druck anliegen, siehe Bezugszeichen 41. Falls ein relativ großes Partikel 31 eine Nadel 13 verstopft, steigt der Druck in der Aspirationsleitung stark an, siehe Bezugszeichen 42. Dieser hohe Druck bleibt so lange bestehen, bis die Okklusion aufbricht, siehe Bezugszeichen 43, wobei sich anschließend in relativ kurzer Zeit der Unterdruck abbaut, siehe Bezugszeichen 44. Durch die starke Sogwirkung kann der Unterdruck sogar in einen Überdruck übergehen, siehe Bezugszeichen 45, wobei er sich anschließend wieder auf einen ursprünglichen Druckpegel einschwenkt, siehe Bezugszeichen 46.

Ein Partikel 31, welches einen derartigen Druckverlauf bewirkt, kann eine Geschwindigkeit aufweisen, wie sie in Figur 4 in dem unteren Diagramm 50 dargestellt ist. Während des normalen Ansaugvorganges, siehe Bezugszeichen 41, kann ein Partikel mit einer normalen Ansauggeschwindigkeit 51 zur Nadel hin angesaugt werden. Tritt eine Okklusion auf, fällt die Geschwindigkeit des Partikels abrupt ab, siehe Bezugszeichen 52, so dass dieses vor einer Nadel nur noch zittert oder vollkommen an der Nadel still verharrt, siehe Bezugszeichen 56. Der Betrag der Geschwindigkeit unterschreitet somit den Betrag eines vorher gesetzten Schwellwertes W1, siehe Fig. 4. Eine solche Änderung der Geschwindigkeit tritt sehr schnell auf und ist ein Zeichen für eine eingetretene Okklusion. Während der Okklusion bis zum Okklusionsdurchbruch kann die Geschwindigkeit des Partikels unverändert sehr niedrig sein. Erst wenn die Okklusion aufbricht, siehe Bezugszeichen 57, kann die Geschwindigkeit der sich dann bildenden kleineren Partikel vor der Nadelspitze wieder ansteigen, siehe Bezugszeichen 53, den Betrag des Schwellwertes W1 überschreiten, und weiter fortwährend ansteigen, siehe Bezugszeichen 54, bis es ein Maximum erreicht, siehe Bezugszeichen 55, um dann langsam wieder auf den Wert vor der Okklusion abzusinken.

Die kinematische Messgröße Geschwindigkeit eines Partikels korreliert somit unmittelbar und direkt mit einer Okklusion innerhalb der Aspirationsleitung, wobei anhand der absoluten Geschwindigkeit oder der Geschwindigkeitsänderung des Partikels der Beginn einer Okklusion und das Aufbrechen einer Okklusion zuverlässig erkannt werden kann. Der Vorteil beim Erfassen einer kinematischen Messgröße Geschwindigkeit im Vergleich zum Aufzeichnen eines Druckverlaufes in einer Aspirationsleitung besteht darin, dass sich beim Beginn einer Okklusion die kinematische Messgröße Geschwindigkeit sehr viel schneller von einem hohen auf einen niedrigen Wert ändert, als bis sich eine Druckänderung in einer Aspirationsleitung einstellt. Dies lässt sich damit erklären, dass das ruhende Partikel vor der Nadel die Ursache für eine Okklusion ist, wohingegen die Druckänderung die Wirkung für eine Okklusion ist. Wenn die Ursache durch ein optisches System ermittelt werden kann, führt dies zu einer schnelleren Detektion einer Okklusion, als wenn die sich langsam aufbauende Druckänderung zur Steuerung herangezogen wird. Dies gilt auch in umgekehrter Weise. Wenn eine Okklusion aufbricht, ändert sich die Geschwindigkeit der Partikel vor der Nadel sehr schnell. Zwar ändert sich dann auch der Druckverlauf in der Aspirationsleitung sehr schnell, jedoch ist dies wieder nur die Folge der frei gewordenen Nadelspitze. Die schnellere Änderung der kinematischen Messgröße Geschwindigkeit im Vergleich zu einer Druckänderung und zusätzlich das Erfassen dieser kinematischen Messgröße mittels eines optischen Systems ermöglicht es, wesentlich schneller als bisher eine Okklusion oder einen Okklusionsdurchbruch zu erfassen.

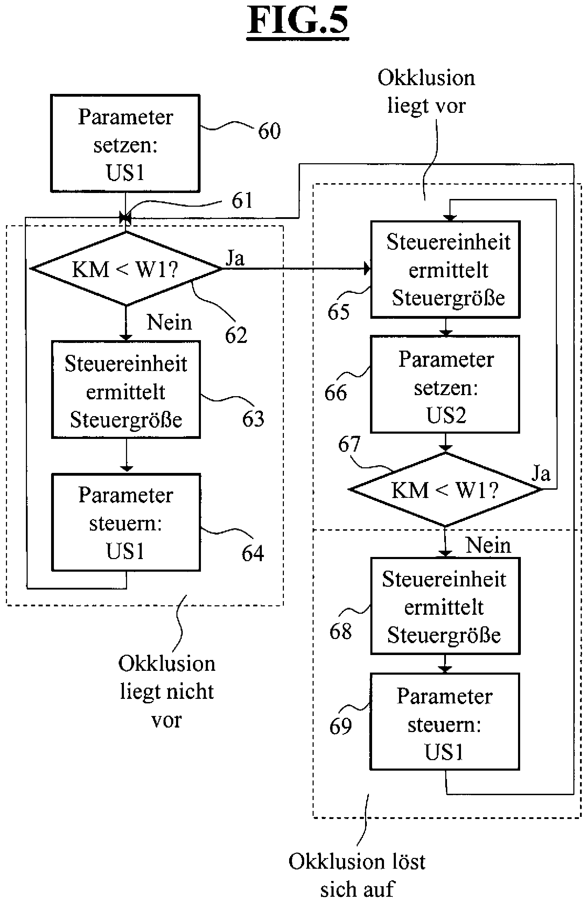

In Figur 5 ist ein Flussdiagramm dargestellt, mit dem die Wechselwirkung beim Erfassen einer kinematischen Messgröße als Auswertegröße und einem zugehörigen Parameter eines ophthalmochirurgischen Systems dargestellt ist. Als Parameter des ophthalmochirurgischen Systems ist eine Ultraschallenergie gewählt, welche dem Phakohandstück zur Emulsifikation von Linsenmaterial zugeführt wird. Zu Beginn wird der Parameter Ultraschallenergie auf einen Betrag US1 gesetzt, siehe Bezugszeichen 60. Bei diesem Betrag kann es sich um eine relativ geringe Energie handeln, so dass nur eine geringe oder keine Emulsifikation eines Linsenmaterials erreicht wird. Wenn als Auswertegröße eine kinematische Messgröße KM, zum Beispiel eine Geschwindigkeit eines Partikels, gewählt wird, kann anschließend abgefragt werden, ob dieser Betrag der kinematischen Messgröße KM kleiner als ein Schwellwert W1 ist. Wenn dies nicht zutrifft, wenn sich also ein Partikel mit relativ hoher Geschwindigkeit bewegt, kann die Steuereinheit eine Steuergröße daraus ermitteln, siehe Kasten 63. Die Steuergröße kann dann an eine Systemkomponenteneinheit zugeführt werden, zum Beispiel eine Energieversorgung 110, so dass der Parameter Ultraschallenergie weiterhin auf dem Wert US1 gesetzt bleibt, siehe Kasten 64. Anschließend kann die Abfrage wieder zu einem Kontenpunkt 61 zurückkehren, wobei gemäß Abfrage 62 erneut gefragt wird, ob die kinematische Messgröße KM kleiner als der Schwellwert W1 ist.

Falls die kinematische Messgröße KM kleiner als der Schwellwert W1 ist, bewegt sich das Partikel offenbar kaum noch oder gar nicht mehr, sodass eine Okklusion erkannt wird. Daraus kann eine Bildauswerteeinheit eine Auswertegröße 8 generieren, welche zur Steuereinheit 9 geleitet wird, welche daraufhin eine zugehörige Steuergröße 10 ermittelt, siehe Kasten 65. Die Steuergröße 10 kann dann auf den Parameter Ultraschallenergie derart einwirken, dass ein höherer Betrag US2 gesetzt wird, siehe Kasten 66. Wenn eine Okklusion vorliegt, kann somit eine höhere Ultraschallenergie angelegt werden, um ein offenbar relativ großes Partikel möglichst schnell zu zertrümmern. Anschließend kann abgefragt werden, ob der momentane Betrag der kinematische Messgröße KM wieder kleiner als der Schwellwert W1 ist, siehe Kasten 67. Wenn also die Geschwindigkeit eines Partikels immer noch relativ niedrig ist und sich dessen Betrag unterhalb des Schwellwertes W1 befindet, liegt offenbar immer noch eine Okklusion vor, so dass die Abfrage erneut zu Kasten 65 geleitet wird. Bei der offenbar vorhandenen Okklusion ermittelt dann eine Steuereinheit 9 erneut eine zugehörige Steuergröße 10, siehe Kasten 65, sodass erneut ein relativ hoher Betrag einer Ultraschallenergie US2 gesetzt wird, siehe Kasten 66. Falls bei der nachfolgenden Abfrage erkannt wird, dass der Betrag der kinematischen Messgröße KM nicht mehr kleiner als der gesetzte Schwellwert W1 ist, liegt offenbar die Situation vor, dass sich eine Okklusion auflöst. Das Partikel oder die Partikel beginnen offenbar, sich mit einer relativ hohen Geschwindigkeit zu bewegen. In diesem Fall kann die Steuereinheit 9 eine zugehörige Steuergröße 10 ermitteln, siehe Kasten 68, und den Parameter Ultraschallenergie wieder auf einen niedrigen Betrag US1 setzten, siehe Kasten 69. Bei einem Okklusionsdurchbruch kann somit die Schwingung der Nadel sofort auf einen niedrigen Betrag reduziert werden. Anschließend wird das Verfahren wieder an Knotenpunkt 61 fortgesetzt, wobei erneut gefragt wird, ob der Betrag einer kinematischen Messgröße KM kleiner als der Schwellwert W1 ist, siehe Kasten 62.

Bei den Kästen 62, 63 und 64 liegt keine Okklusion vor, bei den Kästen 65, 66 und 67 beginnt eine Okklusion sich aufzubauen oder weiterhin zu bestehen, während sich bei den Kästen 68 und 69 eine Okklusion wieder auflöst. Nur durch Erfassen einer kinematischen Messgröße kann somit festgestellt werden, ob eine Okklusion vorliegt oder sich die Okklusion wieder auflöst.

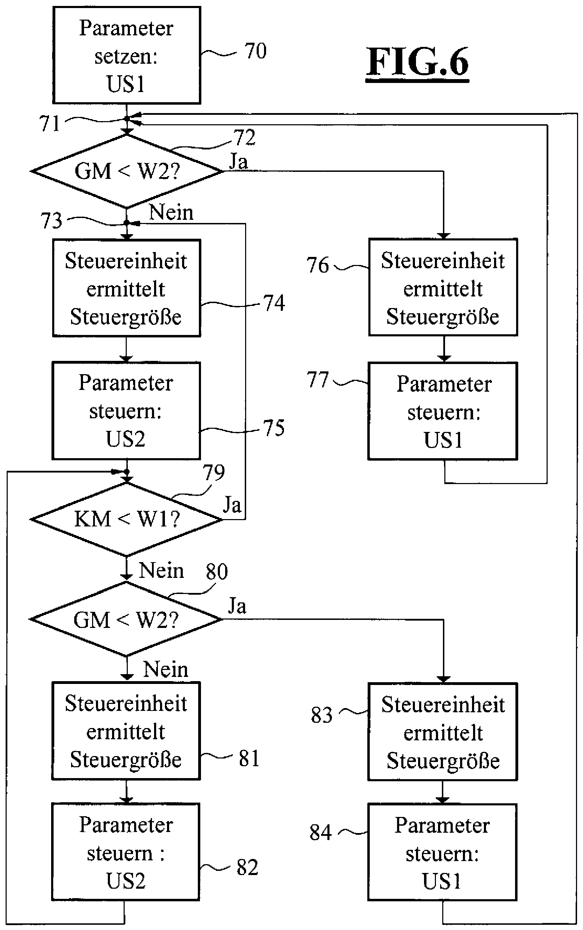

In Figur 6 ist ein Flussdiagramm dargestellt, bei dem eine kinematische Messgröße KM und eine geometrische Messgröße GM genutzt werden, um einen Parameter Ultraschallenergie zu steuern. Zu Beginn wird ein Parameter Ultraschallenergie auf einen Betrag US1 gesetzt, siehe Kasten 70. Anschließend wird abgefragt, ob eine geometrische Messgröße GM kleiner als ein Schwellwert W2 ist. Die geometrische Messgröße kann zum Beispiel eine Fläche eines Partikels sein. Falls die geometrische Messgröße GM kleiner als der Schwellwert W2 ist, also ein relativ kleines Partikel vorliegt, wird von der Auswerteeinheit eine Auswertegröße 8 bestimmt, welche einer Steuereinheit 9 zugeführt wird, die daraufhin eine zugehörige Steuergröße 10 ermittelt, siehe Kasten 76. Da das erfasste Partikel klein genug ist, muss nichts zusätzlich emulsifiziert werden, so dass der Parameter Ultraschallenergie auf dem niedrigen Betrag US1 gesetzt bleiben kann, siehe Kasten 77. Anschließend kann die Abfrage wieder zum Knotenpunkt 71 zurückgeführt werden, so dass wieder abgefragt wird, ob die geometrische Messgröße GM kleiner als ein Schwellwert W2 ist, siehe Kasten 72.

Falls die geometrische Messgröße GM jedoch größer als der Schwellwert W2 ist, also ein relativ großes Teil vor der Nadel 13 vorhanden ist, wird von der Auswerteinheit eine Auswertegröße 8 bestimmt, welche einer Steuereinheit 9 zugeführt wird, die daraufhin eine zugehörige Steuergröße 10 ermittelt, siehe Kasten 74. Der Parameter Ultraschallenergie kann dann auf einen höheren Betrag US2 gesetzt werden, siehe Kasten 75. In diesem Fall kann nur durch das Erfassen einer geometrischen Messgröße der Parameter Ultraschallenergie von einem niedrigen Betrag auf einen höheren Betrag gesetzt werden. Der höhere Betrag US2 ist in diesem Fall sinnvoll, weil bei einem großen Partikel die Gefahr besteht, dass dieses Partikel nicht ohne Verstopfung durch die Aspirationsleitung geführt werden kann. Mit einem höheren Energiebetrag kann dieses große Partikel in kleinere Partikel zertrümmert werden.

Nach Setzen des Parameters auf einen höheren Betrag US2 kann unmittelbar danach abgefragt werden, ob die kinematische Messgröße KM kleiner als der Schwellwert W1 ist. Damit wird geprüft, ob zum Beispiel eine Geschwindigkeit des erkannten Partikels kleiner als der Schwellwert W1 ist, siehe Kasten 79. Falls dies nicht zutrifft, sich das Partikel also mit einer recht hohen Geschwindigkeit bewegt, kann unmittelbar danach gefragt werden, ob eine geometrische Messgröße kleiner als ein Schwellwert W2 ist, siehe Kasten 80. Falls dies zutrifft, und ein Partikel mit einer relativ großen Geschwindigkeit erfasst wird, scheint das Partikel in dem Fluid wieder zu schwimmen.

Durch Abfragen der geometrischen Messgröße wird überprüft, ob das Partikel tatsächlich zu mehreren kleineren Partikeln emulsifiziert worden ist oder nicht. Falls die geometrische Messgröße größer als ein Schwellwert W2 ist, also immer noch ein großes Partikel vorliegt, welches sich lediglich vor der Nadel vor und zurück bewegt oder vor der Nadel zittert und eine Verstopfung immer noch nicht aufgelöst ist, wird von der Steuereinheit eine zugehörige Steuergröße ermittelt, siehe Kasten 81. Da das Partikel immer noch relativ groß zu sein scheint, wird der Parameter Ultraschallenergie immer noch auf einen hohen Betrag US2 belassen, siehe Kasten 82, so dass das relativ große Partikel in kleine Partikel emulsifziert werden kann. Anschließend geht die Abfrage zurück einem Knotenpunkt 78 vor der Abfrage gemäß Kasten 79.

Falls bei der Abfrage 80 der geometrischen Messgröße aber erkannt wird, dass die geometrische Größe kleiner als ein Schwellwert ist, so dass also nur noch kleine Partikelgrößen vorliegen, ermittelt die Steuereinheit 9 eine zugehörige Steuergröße 10, siehe Kasten 83, so dass der Parameter Ultraschallenergie auf einen niedrigen Betrag US1 gesetzt wird, siehe Kasten 84. Wenn also nur noch relativ kleine Partikel vorliegen, ist es nicht mehr erforderlich, weiterhin eine hohe Ultraschallenergie in das System einzubringen. Die dann vorhandenen kleinen Partikel und das zugehörige Fluid können abgesaugt werden, ohne dass weiterhin eine hohe Energie in das System eingebracht wird. Anschließend kann die Abfrage wieder zum Knotenpunkt 71 zurückgeführt werden, so dass wieder abgefragt wird, ob die geometrische Messgröße GM kleiner als ein Schwellwert W2 ist, siehe Kasten 72. Der Ablauf verläuft dann wie vorstehend beschrieben.

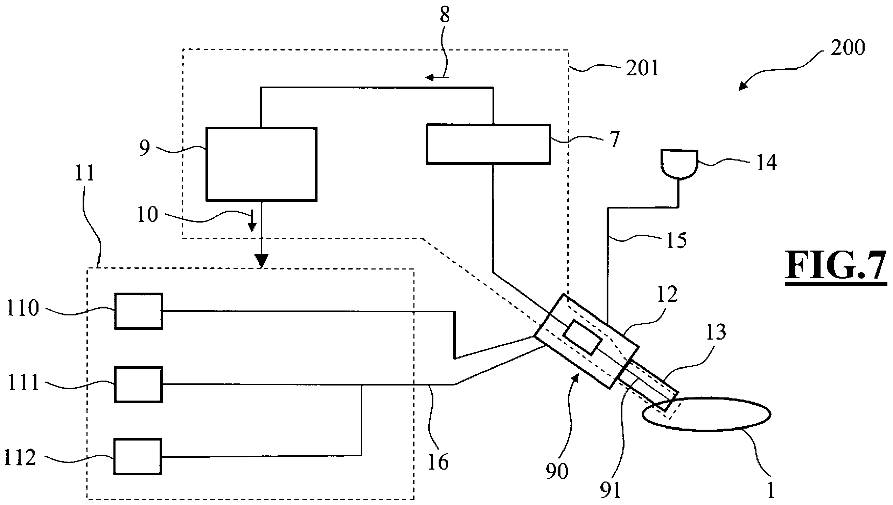

In Figur 7 ist eine zweite Ausführungsform einer Steuerungsvorrichtung für ein ophthalmochirurgischen System dargestellt. Das ophthalmochirurgischen System 200 weist eine Steuerungsvorrichtung 201 auf, welche ein optisches System 90, eine Bildauswerteeinheit 7 und eine Steuereinheit 9 aufweist. Das optische System 90 kann in Form eines OCT-Systems vorliegen, welches eine Quelle für ein zeitlich inkohärentes und räumlich kohärentes Licht aufweist, zusätzlich eine Vorrichtung zur Überlagerung von Laserstrahlung aus einem Probenstrahlengang mit Laserstrahlung aus einem Referenzstrahlengang aufweist, und ferner eine Bilderzeugungseinheit aufweist, um ein Bild zu erzeugen. Zu dem optischen System 90 gehört ferner noch eine OCT-Faser 91, welche in der Nadel 13 oder an der Nadel 13 angeordnet ist. Das von der Bilderzeugungseinheit erzeugte Bild wird daraufhin zu einer Bildauswerteeinheit 7 geleitet, welche das Bild auswertet, wobei eine Auswertegröße 8 ermittelt wird. Diese Auswertegröße 8 kann einer Steuereinheit 9 zugeführt werden, welche eine Steuergröße 10 ermittelt. Die Steuergröße 10 kann anschließend auf eine Systemkomponenteneinheit 11 einwirken, um einen Betrag eines Parameters des ophthalmochirurgischen Systems 200 zu steuern. Die Systemkomponenteneinheit 11 kann zum Beispiel eine Energieversorgung 110, eine Aspirationspumpe 111 oder einen Refluxbehälter 112 aufweisen. Die Systemkomponenteneinheit 11 ist mit einem Phakohandstück 12 gekoppelt, so dass das Signal zum Beispiel von der Energieversorgung 110 zur Ansteuerung einer Phakonadel 13 genutzt werden kann. Das ophthalmochirurgische System 200 weist ferner einen Irrigationsbehälter 14 auf, aus welchem durch eine Irrigationsleitung 15 Fluid zum Handstück 12 und dort zu einer zu behandelnden Augenlinse 1 geleitet werden kann.

Es ist ferner denkbar, dass ein ophthalmochirurgischen System ein optisches System 3 in Form eines Lichtmikroskops und ein optisches System 90 in Form eines OCT-Systems aufweist.

Gemäß einer weiteren Ausführungsform kann auch innerhalb des Lichtmikroskopes ein OCT-System angeordnet sein, wobei zusätzlich innerhalb eines Handstückes ein derartiges OCT-System angeordnet sein kann.

## Patentansprüche

1. Steuerungsvorrichtung (101; 201) für ein ophthalmochirurgisches System (100; 200) zur Phakoemulsifikation einer Augenlinse (1), aufweisend:
- ein optisches System (3; 90), mit dem von einem Objektbereich (2) ein Bild erzeugbar ist, wobei sich in dem Objektbereich (2) mindestens ein Teil der zu emulsifizierenden Augenlinse (1) und ein Teil einer Nadel (13) eines Phakohandstückes (12) mit einer Aspirationsleitung (16) anordnen lässt, **gekennzeichnet dadurch, dass** sie aufweist:
- eine Bildauswerteeinheit (7), welche eingerichtet ist, das erzeugte Bild (20; 22) so auszuwerten, dass mindestens eine von einer Okklusion der Aspirationsleitung (16) abhängige Auswertegröße (8) ermittelt wird, welche eine kinematische Messgröße und/oder eine geometrische Messgröße und/oder eine optische Messgröße eines durch Emulsifikation erzeugten Partikels (21, 31) der zu behandelnden Augenlinse (1) ist,
- eine Steuereinheit (9), welche eingerichtet ist, in Abhängigkeit von der mindestens einen zugeführten Auswertegröße (8) eine Steuergröße (10) zu ermitteln und mittels der Steuergröße (10) einen Betrag eines Parameters des ophthalmochirurgischen Systems (100; 200) zu steuern.

2. Steuerungsvorrichtung (101; 201) nach Anspruch 1, wobei die kinematische Messgröße die Geschwindigkeit oder Beschleunigung des Partikels (21, 31) während einer vorbestimmten Zeitdauer ist.

3. Steuerungsvorrichtung (101; 201) nach Anspruch 1 oder 2, wobei die geometrische Messgröße die Fläche oder der Umfang oder das Volumen des zu emulsifizierenden Partikels (21, 31) in dem erzeugten Bild ist.

4. Steuerungsvorrichtung (101; 201) nach einem der vorherigen Ansprüche, wobei die optische Messgröße eine Grauwertintensität oder eine Farbintensität ist.

5. Steuerungsvorrichtung (101; 201) nach einem der Ansprüche 1 bis 4, wobei die mindestens eine Auswertegröße (8) der Bildauswerteeinheit (7) eine geometrische Messgröße eines die Nadel (13) umgebenden Gewebes ist.

6. Steuerungsvorrichtung (101; 201) nach einem der vorherigen Ansprüche, wobei der Parameter des ophthalmochirurgischen Systems (100; 200) eine dem Phakohandstück (12) durch eine Energieversorgung (110) zugeführte Ultraschallenergie, eine Saugleistung einer Aspirationspumpe (111) oder ein Volumenstrom eines Fluides zur Belüftung einer Aspirationsleitung ist.

7. Steuerungsvorrichtung (101; 201) nach einem der vorherigen Ansprüche, wobei das optische System (3; 90) ein Lichtmikroskop (3) und/oder ein OCT-System (90) aufweist.

8. Steuerungsvorrichtung (101; 201) nach einem der vorherigen Ansprüche, wobei das optische System (90) mindestens teilweise im Phakohandstück (12) angeordnet ist.

9. Ophthalmochirurgisches System (100; 200), welches eine Steuerungsvorrichtung (101; 201) nach einem der vorherigen Ansprüche aufweist.

## Claims

1. Control device (101; 201) for an ophthalmic surgical system (100; 200) for phacoemulsification of an eye lens (1), having:
- an optical system (3; 90), by means of which an image can be generated of an object region (2), wherein at least part of the eye lens (1) to be emulsified and part of a needle (13) of a phacoemulsification handpiece (12) with an aspiration line (16) can be arranged in the object region (2),
**characterized in that** said control device has:
- an image evaluation unit (7), which is configured for evaluating the generated image (20; 22) in such a way that at least one evaluation variable (8), dependent on an occlusion of the aspiration line (16), is established, which is a kinematic measurement variable and/or a geometric measurement variable and/or an optical measurement variable of a particle (21, 31), produced by emulsification, of the eye lens (1) to be treated,
- a control unit (9), which is configured, dependent on the at least one supplied evaluation variable (8), to establish a control variable (10) and to control an absolute value of a parameter of the ophthalmic surgical system (100; 200) using the control variable (10).

2. Control device (101; 201) according to Claim 1, wherein the kinematic measurement variable is the velocity or acceleration of the particle (21, 31) during a predetermined period of time.

3. Control device (101; 201) according to Claim 1 or 2, wherein the geometric measurement variable is the area or the circumference or the volume of the particle (21, 31) to be emulsified in the generated image.

4. Control device (101; 201) according to one of the preceding claims, wherein the optical measurement variable is a grayscale-value intensity or a color intensity.

5. Control device (101; 201) according to one of Claims 1 to 4, wherein the at least one evaluation variable (8) of the image evaluation unit (7) is a geometric measurement variable of a tissue surrounding the needle (13).

6. Control device (101; 201) according to one of the preceding claims, wherein the parameter of the ophthalmic surgical system (100; 200) is ultrasound energy supplied to the phacoemulsification handpiece (12) by an energy supply (110), a suction power of an aspiration pump (111) or a volumetric flow rate of a fluid for venting an aspiration line.

7. Control device (101; 201) according to one of the preceding claims, wherein the optical system (3; 90) has a light microscope (3) and/or an OCT system (90).

8. Control device (101; 201) according to one of the preceding claims, wherein the optical system (90) is at least partly arranged in the phacoemulsification handpiece (12).

9. Ophthalmic surgical system (100; 200), which has a control device (101; 201) according to one of the preceding claims.

## Revendications

1. Dispositif de commande (101 ; 201) d'un système de chirurgie ophtalmique (100 ; 200) servant à la phacoémulsification d'un cristallin (1), comprenant :
- un système optique (3 ; 90) permettant de produire une image à partir d'une zone (2) d'un objet, dans lequel au moins une partie du cristallin (1) à émulsifier et une partie d'une aiguille (13) d'une pièce à main de phacoémulsification (12) peuvent être disposées dans la zone (2) de l'objet par une conduite d'aspiration (16), **caractérisé en ce qu'**il comprend :
- une unité d'évaluation d'image (7) qui est conçue pour évaluer l'image produite (20 ; 22) de manière à déterminer au moins une grandeur d'évaluation (8) dépendant d'une occlusion de la conduite d'aspiration (16), laquelle grandeur est une grandeur de mesure cinématique et/ou une grandeur de mesure géométrique et/ou une grandeur de mesure optique d'une particule (21, 31) du cristallin (1) à traiter, produite par émulsification,
- une unité de commande (9) qui est conçue pour déterminer une grandeur de commande (10) en fonction de ladite grandeur d'évaluation fournie (8) et pour commander au moyen de la grandeur de commande (10) une valeur d'un paramètre du système de chirurgie ophtalmique (100 ; 200).

2. Dispositif de commande (101 ; 201) selon la revendication 1, dans lequel le paramètre cinématique est la vitesse ou l'accélération de la particule (21, 31) pendant une période de temps prédéterminée.

3. Dispositif de commande (101 ; 201) selon la revendication 1 ou 2, dans lequel la grandeur de mesure géométrique est la surface ou la circonférence ou le volume de la particule (21, 31) à émulsifier dans l'image produite.

4. Dispositif de commande (101 ; 201) selon l'une des revendications précédentes, dans lequel la grandeur de mesure optique est une intensité de niveau de gris ou une intensité de couleur.

5. Dispositif de commande (101 ; 201) selon l'une des revendications 1 à 4, dans lequel ladite au moins une grandeur d'évaluation (8) de l'unité d'évaluation d'image (7) est une grandeur de mesure géométrique d'un tissu entourant l'aiguille (13).

6. Dispositif de commande (101 ; 201) selon l'une des revendications précédentes, dans lequel le paramètre du système de chirurgie ophtalmique (100 ; 200) est une énergie ultrasonore appliquée à la pièce à main de phacoémulsification (12) par une alimentation électrique (110), une puissance d'aspiration d'une pompe d'aspiration (111) ou un débit volumique d'un fluide destiné à l'aération d'une conduite d'aspiration.

7. Dispositif de commande (101 ; 201) selon l'une des revendications précédentes, dans lequel le système optique (3 ; 90) comporte un microscope optique (3) et/ou un système OCT (90).

8. Dispositif de commande (101 ; 201) selon l'une des revendications précédentes, dans lequel le système optique (90) est disposé au moins partiellement dans la pièce à main de phacoémulsification (12).

9. Système de chirurgie ophtalmologique (100 ; 200) comprenant un dispositif de commande (101 ; 201) selon l'une des revendications précédentes.
